# EUROPÄISCHE PATENTANMELDUNG

(11) **EP 1 561 484 A1**
(43) Veröffentlichungstag der Anmeldung: **10.08.2005**
(21) Anmeldenummer: 05001530.4
(22) Anmeldetag: 26.01.2005
(51) Int. Cl.: A61M 15/00, B05B 17/06, F04B 43/04

(54) **Mikrodosiervorrichtung**

(30) Priorität: 05.02.2004 DE 102004006452
(71) Anmelder: Ing. Erich Pfeiffer GmbH, 78315 Radolfzell (DE)
(72) Erfinder: Körner, Joachim, 88690 Uhldingen (DE); Helmlinger, Michael, 78315 Radolfzell (DE); Schürle, Holger, 78315 Radolfzell (DE)
(74) Vertreter: Patentanwälte Ruff, Wilhelm, Beier, Dauster & Partner

(57) **Zusammenfassung**

2.1 Eine Mikrodosiervorrichtung mit einem Dosierraum zur zumindest zeitweisen Aufnahme einer Flüssigkeitsmenge, dem wenigstens eine Austragöffnung zugeordnet ist, sowie mit einer Vibrationseinheit, die mit wenigstens einer Begrenzungsfläche des Dosierraums in Wirkverbindung steht, um diese für einen Austragvorgang in Schwingungen zu versetzen, ist bekannt.
2.2 Erfindungsgemäß sind an dem Dosierraum zumindest zwei voneinander beabstandete Zulaufkanäle vorgesehen.

## Beschreibung

Die Erfindung betrifft eine Mikrodosiervorrichtung mit einem Dosierraum zur zumindest zeitweisen Aufnahme einer Flüssigkeitsmenge, dem wenigstens eine Austragöffnung zugeordnet ist, sowie mit einer Vibrationseinheit, die mit wenigstens einer Begrenzungsfläche des Dosierraums in Wirkverbindung steht, um diese für einen Austragvorgang in Schwingungen zu versetzen.

Mikrodosiervorrichtungen für die Abgabe von kleinen und kleinsten Tröpfchen sind von kosmetischen oder pharmazeutischen Mediumspendern wie beispielsweise Inhalationsgeräten für flüssige medizinische Substanzen bekannt. Sie weisen einen Dosierraum auf, der aus einem Mediumspeicher mit einer Flüssigkeitsmenge befüllbar ist und der zumindest eine Austragöffnung aufweist, durch die die Flüssigkeit nach Überwindung eines Strömungswiderstandes entweichen kann. Der Strömungswiderstand bestimmt sich insbesondere durch Größe, Geometrie und Material der Austragöffnung sowie durch eine Oberflächenspannung und eine Viskosität der Flüssigkeit. Um diesen Strömungswiderstand zu überwinden, ist an wenigstens einer Begrenzungsfläche des Dosierraums eine Vibrationseinheit vorgesehen, die den Dosierraum in Schwingungen versetzen kann. Durch die Schwingungen bilden sich in der Flüssigkeit Druckwellen aus, die zu einer zumindest zeitweisen Überwindung des Strömungswiderstandes der Austragöffnung führen. Dadurch kann die Flüssigkeit durch die Austragöffnung in eine Umgebung ausgestoßen werden.

Aufgabe der Erfindung ist es, eine Mikrodosiervorrichtung der eingangs genannten Art zu schaffen, bei der eine zuverlässige Versorgung des Dosierraums mit der auszutragenden Flüssigkeit gewährleistet ist.

Diese Aufgabe wird dadurch gelöst, dass an dem Dosierraum zumindest zwei voneinander beabstandete Zulaufkanäle vorgesehen sind. Zulaufkanäle bilden eine kommunizierende Verbindung zwischen dem Dosierraum und vorgeschalteten mediumführenden Komponenten wie einer insbesondere als Mediumpumpe ausgeführten Befüllungseinrichtung, die auch als Kapillarrohr oder als Druckbehältersystem mit Steigrohr gestaltet sein und eine Verbindung zu einem Mediumspeicher herstellen kann. Die Zulaufkanäle erlauben damit ein Einströmen der Flüssigkeit aus dem Mediumspeicher in den Dosierraum. Bei Vorliegen eines einzigen Zulaufkanals an dem Dosierraum, wie aus dem Stand der Technik bekannt, ist eine gleichmäßige Versorgung des gesamten Dosierraums mit Flüssigkeit während eines Austragvorgangs häufig nicht gewährleistet. Mit bekannten Mikrodosiervorrichtungen sind Anforderungen, wie sie in vielen Anwendungsfällen gestellt werden, allenfalls zufriedenstellend zu erfüllen.

Für erhöhte Anforderungen an eine Dosiergenauigkeit und/oder an eine Verteilung ausgetragener Tröpfchen der Flüssigkeit sind bekannte Mikrodosiervorrichtungen nur bedingt geeignet. Der Dosierraum gemäß der Erfindung, an dem zumindest zwei voneinander beabstandete Zulaufkanäle vorgesehen sind, ermöglicht hingegen bei gleichzeitiger Erhöhung der Dosiergenauigkeit und Verbesserung der Verteilung der Tröpfchen eine Reduktion der Herstellungskosten für die Mikrodosiervorrichtung. Die Herstellungskosten für eine Mikrodosiervorrichtung werden insbesondere von einer Dimensionierung von Fördermitteln, die zur Förderung der Flüssigkeit aus dem Mediumspeicher hin zum Dosierraum eingesetzt werden, beeinflusst. Durch mehrere, voneinander beabstandete Zulaufkanäle kann eine homogenere Versorgung des Dosierraums mit der auszutragenden Flüssigkeit gewährleistet werden. Während des Austragvorgangs können lokale Flüssigkeitsdefizite, die zu einer Effizienzreduktion der Mikrodosiervorrichtung führen könnten, vermieden werden. Dadurch kann eine kleinere Dimensionierung und/oder ein einfacherer Aufbau der Fördermittel und damit eine Senkung der Herstellungskosten für die Mikrodosiervorrichtung erreicht werden. Ein weiterer Vorteil mehrerer Zulaufkanäle liegt darin, dass ein für den Austrag einer definierten Flüssigkeitsmenge benötigter Energiebedarf durch gesteigerte Effizienz des Dosierraums reduziert werden kann. Dadurch kann ein zur Versorgung der Vibrationseinheit vorzusehender Energiespeicher kleiner dimensioniert werden, wodurch sich zusätzliche Herstellungskosten einsparen lassen. Die Zulaufkanäle können für eine gute Befüllung des Dosierraums sowohl mit einer einheitlichen Ausrichtung an einer einzigen Begrenzungsfläche des Dosierraums als auch mit unterschiedlichen Ausrichtungen an einer oder mehreren Begrenzungsflächen des Dosierraums vorgesehen sein. Die Vibrationseinheit kann mit einem oder mehreren Mitteln zur Schwingungserzeugung versehen sein. Diese sind insbesondere als Piezokristallschwinger ausgeführt. Die Mittel zur Schwingungserzeugung bewirken eine Anregung der Begrenzungsfläche in einer oder mehreren gleich- oder verschiedenartig ausgerichteten, gleichphasig oder phasenversetzt wirkenden Schwingungsrichtungen.

In Ausgestaltung der Erfindung ist zumindest eine Vorratskammer mit dem Dosierraum über zumindest einen Zulaufkanal kommunizierend verbunden. Eine Vorratskammer ist für eine temporäre Speicherung von auszutragender Flüssigkeit vorgesehen, insbesondere um Spitzenfördermengen während des Austragsvorgangs ausgleichen zu können. Dadurch sorgt die Vorratskammer auch für eine weitere Homogenisierung der Verteilung der Tröpfchen und für eine Erhöhung der Dosiergenauigkeit. Die Vorratskammer ist dazu über zumindest einen Zulaufkanal kommunizierend mit dem Dosierraum verbunden, so dass Flüssigkeit, die in der Vorratskammer gespeichert ist, unmittelbar über den Zulaufkanal in den Dosierraum einströmen kann. Die Vorratskammer kann sowohl passiv ohne eigene Fördereinrichtung als auch aktiv mit eigener Fördereinrichtung ausgeführt sein. Eine Befüllung der Vorratskammer kann lediglich zeitweilig, insbesondere unmittelbar vor einem Austragvorgang, oder auch permanent vorgesehen sein.

In weiterer Ausgestaltung der Erfindung ist die Vorratskammer an einem der Dosierkammer abgewandten Endbereich mit einer Ausgleichsöffnung versehen. Durch eine Ausgleichsöffnung an der Vorratskammer wird ein Druckaufbau in der Vorratskammer während einer Befüllung mit Flüssigkeit vermindert oder vermieden. Weiterhin wird durch die Ausgleichsöffnung ein Abströmen der Flüssigkeit in Richtung des Dosierraums erleichtert, da durch die Ausgleichsöffnung ein Druckausgleich gegenüber einer Umgebung stattfinden kann. Damit stellt die Vorratskammer einen geringen Widerstand für die Flüssigkeit dar. Eine Vorratskammer kann insbesondere als Kapillare ausgeführt werden, wodurch sich bedingt durch die Kapillarkräfte eine besonders einfache Befüllung erzielen läßt. Weiterhin ist bei einer Kapillare eine vollständige Befüllung und Entleerung ohne Gasblasen in der Flüssigkeit in einfacher Weise zu erzielen, so dass die bevorratete Flüssigkeit unmittelbar und insbesondere ohne Maßnahmen zur Gasabscheidung in den Dosierraum eingeleitet werden kann.

In weiterer Ausgestaltung der Erfindung ist die Ausgleichsöffnung der Vorratskammer mit einem Gasfilter verschlossen. Durch einen Gasfilter, der insbesondere als hydrophober Membranfilter ausgeführt sein kann, kann eine Kontamination der in der Vorratskammer gespeicherten Flüssigkeit durch Keime aus einer Umgebung der Mikrodosiervorrichtung verhindert werden. Der Gasfilter ist dazu so ausgelegt, dass er lediglich Gaspartikel passieren lässt, während Bakterien, Viren oder Flüssigkeit am Durchtritt durch den Gasfilter gehindert werden. Damit kann der Gasfilter zusätzlich auch als Füllbegrenzung für die Vorratskammer genutzt werden, da ein Austreten der Flüssigkeit aus der Vorratskammer in die Umgebung ausgeschlossen werden kann. Der Filter kann dabei insbesondere als diskretes Bauteil an der Vorratskammer angebracht werden oder, wie in einer bevorzugten Ausführungsform der Erfindung, als Filterpatrone in die Ausgleichsöffnung der Vorratskammer eingebracht werden.

In weiterer Ausgestaltung der Erfindung ist zumindest ein Zulaufkanal mit einem Mediumleiter verbunden und zumindest eine Verbindungskapillare zwischen dem Mediumleiter und der Vorratskammer vorgesehen. Ein Mediumleiter stellt eine kommunizierende Verbindung zwischen Mediumspeicher und Dosierraum her und ist zu diesem Zweck mit zumindest einem Zulaufkanal verbunden. Um eine vorteilhafte Verteilung der Flüssigkeit in unmittelbarer Umgebung des Dosierraums zu erreichen, ist zumindest eine Verbindungskapillare zwischen dem Mediumleiter und der Vorratskammer vorgesehen. Durch die Verbindungskapillare kann eine direkte Versorgung der Vorratskammer mit Flüssigkeit aus dem Mediumspeicher gewährleistet werden. Eine parallele Versorgung der Vorratskammer über den Dosierraum ist weiterhin möglich.

In weiterer Ausgestaltung der Erfindung weist ein durch die Verbindungskapillare, die Vorratskammer und den Gasfilter gebildeter Mediumzweig einen geringeren Strömungswiderstand als die zumindest eine Austragöffnung auf. Der Strömungswiderstand der Austragöffnung wird, wie bereits ausgeführt, insbesondere durch Größe, Geometrie und Material der Austragöffnung und die Oberflächenspannung der Flüssigkeit bestimmt. Sofern ein aus der Verbindungskapillare der Vorratskammer und dem Gasfilter gebildeter Mediumzweig einen geringeren Strömungswiderstand als die Austragöffnung aufweist, kann während einer Befüllung der Vorratskammer über aktive Fördermittel ein unerwünschtes Austreten von Flüssigkeit durch die Austragöffnung verhindert werden.

In weiterer Ausgestaltung der Erfindung ist in der Vorratskammer eine Messeinrichtung zur Durchfluss- und/oder Füllstandsermittlung vorgesehen. Eine Messeinrichtung kann insbesondere als kapazitiver, induktiver oder optischer Durchflussmesser bzw. Volumenmesser ausgeführt werden. Durch eine derartige Messeinrichtung kann insbesondere sichergestellt werden, dass ein Austragvorgang erst dann auslösbar ist, sobald die Vorratskammer mit einer Mindestfüllmenge befüllt ist. Damit können die Vorteile der Vorratskammer optimal ausgenutzt werden. Eine Ermittlung eines Durchflussmesswertes durch die Messeinrichtung in der Vorratskammer ermöglicht eine optimierte Anpassung einer Förderleistung der aktiven Fördermittel und/oder der Vibrationseinheit, um eine energieeffiziente und homogene Fördermenge der Mikrodosiervorrichtung zu gewährleisten. Ein Abströmen der Flüssigkeit aus der Vorratskammer kann weiterhin als direktes oder indirektes Maß für eine aus dem Dosierraum ausgetragene Flüssigkeitsmenge herangezogen werden.

In weiterer Ausgestaltung der Erfindung ist die Vorratskammer zumindest abschnittsweise gemeinsam mit dem Mediumleiter in einem Bauteil vorgesehen. Das Bauteil kann insbesondere aus Silizium, Kunststoff, Metall oder Verbundwerkstoff hergestellt sein. Bei der Herstellung aus Kunststoff ergibt sich eine besonders kostengünstige Gestaltung. In einer besonders bevorzugten Ausführungsform ist die Vorratskammer mit dem Mediumleiter, den Zulaufkanälen und zumindest einer Begrenzungsfläche des Dosierraums in einem einzigen gemeinsamen Bauteil aus Kunststoff verwirklicht.

In weiterer Ausgestaltung der Erfindung ist in dem Bauteil eine Befüllungseinrichtung, insbesondere eine Mediumpumpe zur Beförderung der Flüssigkeit durch den Mediumleiter in die Vorratskammer und in die Dosierkammer vorgesehen. Eine Mediumpumpe dient als aktives Fördermittel zum Transport der Flüssigkeit vom Mediumspeicher zur Vorratskammer und zur Dosierkammer. Durch eine Integration der Mediumpumpe in das Bauteil, in dem auch die Vorratskammer und die Dosierkammer sowie der Mediumleiter vorgesehen sein können, lässt sich eine besonders kompakte und kostengünstige Baugruppe für eine Mikrodosiervorrichtung erzielen. In einer besonders bevorzugten Ausführungsform kann die Vibrationseinheit, die ohnehin für eine Anregung einer Begrenzungsfläche des Dosierraums vorgesehen ist, zusätzlich noch als Antrieb der Mediumpumpe eingesetzt werden. Damit ist eine extrem gute Energieeffizienz der Mikrodosiervorrichtung gewährleistet.

In weiterer Ausgestaltung der Erfindung sind die Zulaufkanäle mit unterschiedlichen Strömungswiderständen ausgestattet. In Abhängigkeit der Ausführung des Mediumleiters, der Vorratskammer, der Dosierkammer und der Zulaufkanäle können sich unterschiedliche Strömungswiderstände zwischen dem Mediumleiter bzw. der Befüllungseinrichtung und den einzelnen in den Dosierraum mündenden Zulaufkanälen ergeben. Um dennoch für eine homogene Versorgung des Dosierraums aus allen vorgesehenen Zulaufkanälen zu sorgen, können diese durch geometrische Ausgestaltung und/oder entsprechende Anordnung am Dosierraum bzw. spezielle Beschichtungen in ihrem Strömungswiderstand so eingestellt werden, dass sich bezogen auf den Mediumleiter bzw. auf die Befüllungseinrichtung für alle Zuleitungszweige ein im wesentlichen identischer Strömungswiderstand ergibt.

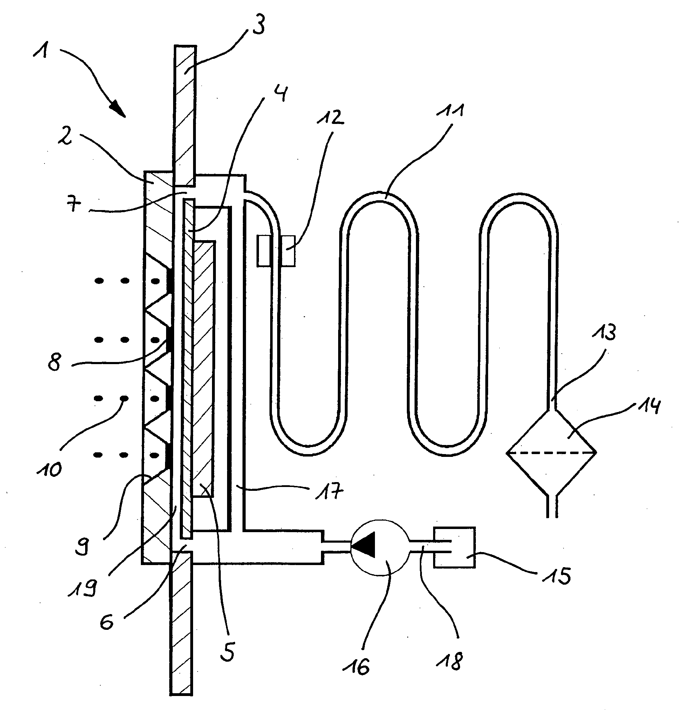

Weitere Vorteile und Merkmale der Erfindung ergeben sich aus den Ansprüchen sowie aus der Beschreibung eines bevorzugten Ausführungsbeispiels der Erfindung, das anhand der einzigen Figur dargestellt ist.

Die einzige Figur zeigt in ebener schematischer Darstellung eine Mikrodosiervorrichtung mit Dosierraum und Vorratskammer.

Eine Mikrodosiervorrichtung 1 weist einen als Kompressionskammer 19 ausgeführten Dosierraum auf, der von Begrenzungsflächen wie einer Membran 2, einem Bauteil 3 und einer Rückwand 4 begrenzt ist. In der Membran 2 sind nicht näher dargestellte, als Membranporen 8 ausgeführte Austragöffnungen vorgesehen, durch die eine in der Kompressionskammer zumindest zeitweilig enthaltene Flüssigkeit in eine Umgebung der Mikrodosiervorrichtung 1 ausgebracht werden kann. An der Rückwand 4 der Kompressionskammer 19 ist eine als Ultraschallschwinger 5 ausgeführte Vibrationseinheit angebracht, die durch nicht näher dargestellte Ansteuermittel zumindest zeitweilig mit elektrischer Energie zur Erzeugung einer Schwingung versorgt werden kann. An der Kompressionskammer 19 sind weiterhin mehrere Zulaufkanäle vorgesehen, von denen einer als Direktzufluss 6 und ein weiterer als Speicherzufluss 7 verwirklicht ist. Während der Direktzufluss 6 unmittelbar mit einer Befüllungseinrichtung 16 gekoppelt ist, wird der Speicherzufluss 7 sowohl von einer als Kanal 17 ausgeführten Verbindungskapillare als auch von einem als mäanderförmiger Speicher 11 ausgeführten Vorratskammer mit Flüssigkeit versorgt.

Der Speicher 11 ist als mäanderförmige Kapillarstruktur in einem nicht näher dargestellten Bauteil eingebracht und weist an einem dem Speicherzufluss 7 abgewandten Ende eine als Entlüftung 13 ausgeführte Ausgleichsöffnung auf. Die Entlüftung 13 ist dabei durch einen als hydrophobes Filterelement 14 ausgeführten Gasfilter verschlossen, so dass eine Barriere für Keime und Flüssigkeit am Ende des Speichers 11 vorliegt. Der Kanal 17 ist mit dem Direktzufluss 6 sowie mit der Pumpeinrichtung 16 verbunden. Die Pumpeinrichtung 16 versorgt sich über einen als Steigrohr 18 ausgeführten Mediumleiter aus einem als Vorratsbehälter 15 ausgeführten Mediumspeicher mit Flüssigkeit. Zur Ermittlung einer von der Mikrodosiervorrichtung 1 ausgebrachten Flüssigkeitsmenge ist am Speicher 11 eine als Durchflussmesser 12 ausgeführte Messeinrichtung vorgesehen, die mit einer nicht dargestellten Regeleinheit zur Ansteuerung des Ultraschallschwingers 4 vorgesehen ist.

Für einen Austragvorgang zur präzisen Dosierung einer Flüssigkeitsmenge in eine Umgebung der Mikrodosiervorrichtung 1 ist zunächst vorgesehen, dass Flüssigkeit aus dem Vorratsbehälter 15 über das Steigrohr 18 von der Pumpeinrichtung 16 angesaugt wird und in Richtung des Direktzuflusses 6 und des Speicherzuflusses 7 gefördert wird.

Sofern zu diesem Zeitpunkt der Speicher 11 nicht mit Flüssigkeit gefüllt ist, ergibt sich durch den geringen Strömungswiderstand des aus dem Kanal 17, dem Speicher 11 und dem Filterelement 14 gebildeten Mediumzweigs ein Flüssigkeitsstrom in den Speicher 11. Dabei tritt die Flüssigkeit nicht aus den Membranporen 8 in die Umgebung aus, da der Strömungswiderstand der Membranporen 8 höher als der Strömungswiderstand des Mediumzweigs ist. Sofern der Speicher 11 und die Kompressionskammer 19 mit Flüssigkeit befüllt sind, kann der Austragvorgang durch Aktivierung des Ultraschallschwingers 5 über nicht dargestellte Energieversorgungs- und Ansteuermittel vorgesehen werden, so dass durch Schwingung der Rückwand 4 in der Kompressionskammer 19 ein Druckaufbau stattfindet, der zur Überwindung des Strömungswiderstandes der Membranporen 8 und damit zu einem Austrag der Flüssigkeit in die Umgebung führt.

Nachdem in der Kompressionskammer 19 bedingt durch die Schwingung des Ultraschallschwingers kein statischer, sondern ein oszillierender Druck vorliegt, kann in Phasen geringen Druckes in der Kompressionskammer Flüssigkeit über aktive Förderung durch die Pumpeinrichtung 16 und/oder passives Nachströmen aus dem Speicher 11 in die Kompressionskammer stattfinden. Dadurch ist während des gesamten Austragsvorgangs eine homogene Flüssigkeitsversorgung in der Kompressionskammer sichergestellt, wobei am Ende des Austragvorgangs durchaus auch ein vollständiges Leerlaufen des Speichers 11 und der Kompressionskammer 19 vorgesehen werden kann.

## Patentansprüche

1. Mikrodosiervorrichtung (1) mit einem Dosierraum (19) zur zumindest zeitweisen Aufnahme einer Flüssigkeitsmenge, dem wenigstens eine Austragöffnung (8) zugeordnet ist, sowie mit einer Vibrationseinheit (5), die mit wenigstens einer Begrenzungsfläche (2, 3, 4) des Dosierraums (19) in Wirkverbindung steht, um diese für einen Austragvorgang in Schwingungen zu versetzen, **dadurch gekennzeichnet, dass** an dem Dosierraum (19) zumindest zwei voneinander beabstandete Zulaufkanäle (6, 7) vorgesehen sind.

2. Mikrodosiervorrichtung nach Anspruch 1, **dadurch gekennzeichnet, dass** zumindest eine Vorratskammer (11) mit dem Dosierraum (19) über zumindest einen Zulaufkanal (6, 7) kommunizierend verbunden ist.

3. Mikrodosiervorrichtung nach Anspruch 2, **dadurch gekennzeichnet, dass** die Vorratskammer (11) an einem der Dosierkammer (19) abgewandten Endbereich mit einer Ausgleichsöffnung (13) versehen ist.

4. Mikrodosiervorrichtung nach Anspruch 3, **dadurch gekennzeichnet, dass** die Ausgleichsöffnung (13) der Vorratskammer (11) mit einem, insbesondere als hydrophoben Membranfilter ausgeführten, Gasfilter (14) verschlossen ist.

5. Mikrodosiervorrichtung nach einem der vorhergehenden Ansprüche, **dadurch gekennzeichnet, dass** zumindest ein Zulaufkanal (6, 7) mit einem Mediumleiter (18) verbunden ist und zumindest eine Verbindungskapillare (17) zwischen dem Mediumleiter (18) und der Vorratskammer (11) vorgesehen ist.

6. Mikrodosiervorrichtung nach Anspruch 5, **dadurch gekennzeichnet, dass** ein durch die Verbindungskapillare (17), die Vorratskammer (11) und den Gasfilter (14) gebildeter Mediumzweig einen geringeren Strömungswiderstand als die zumindest eine Austragöffnung (8) aufweist.

7. Mikrodosiervorrichtung nach einem der vorhergehenden Ansprüche, **dadurch gekennzeichnet, dass** in der Vorratskammer (11) eine Meßeinrichtung (12) zur Durchfluß- und/oder Füllstandsermittlung vorgesehen ist.

8. Mikrodosiervorrichtung nach einem der vorhergehenden Ansprüche, **dadurch gekennzeichnet, dass** die Vorratskammer (8) zumindest abschnittsweise gemeinsam mit dem Mediumleiter (18) in einem Bauteil vorgesehen ist.

9. Mikrodosiervorrichtung nach einem der vorhergehenden Ansprüche, **dadurch gekennzeichnet, dass** in dem Bauteil eine Befüllungseinrichtung (16) zur Beförderung der Flüssigkeit durch den Mediumleiter (18) in die Vorratskammer (11) und die Dosierkammer (19) vorgesehen ist.

10. Mikrodosiervorrichtung nach einem der vorhergehenden Ansprüche, **dadurch gekennzeichnet, dass** die Zulaufkanäle (6, 7) mit unterschiedlichen Strömungswiderständen ausgestattet sind.
